# EUROPEAN PATENT APPLICATION

(11) **EP 4 581 940 A1**
(43) Date of publication of application: **09.07.2025**
(21) Application number: 23860619.8
(22) Date of filing: 22.06.2023
(51) Int. Cl.: A23L 29/00, A23L 33/00, C12N 1/20, A61K 35/744, A61P 3/00

(54) **NOVEL LACTIC ACID LACTIPLANTIBACILLUS PLANTARUM SKO-001 BACTERIUM FOR REDUCING BODY FAT, AND USES THEREOFHH**

(30) Priority: 29.08.2022 KR 20220108503
(71) Applicant: Kolmar BNH Co., Ltd, Sejong 30003 (KR)
(72) Inventor: KIM, Seul Ki, Seoul 06798 (KR); SEO, Hee, Seoul 06802 (KR); LEE, Hak Sung, Hwaseong-si, Gyeonggi-do 18478 (KR); KIM, Ju Gyeong, Seoul 06372 (KR); KIM, Sang Back, Seoul 06798 (KR); JU, Jae Yeong, Seongnam-si, Gyeonggi-do 13622 (KR); CHEON, Hyae Gyeong, Seoul 07225 (KR)
(74) Representative: Regimbeau
(86) International application number: PCT/KR2023/008658
(87) International publication number: WO 2024/048934

(57) **Abstract**

According to one embodiment of the present disclosure, the present disclosure relates to a novel probiotic strain of *Lactiplantibacillus plantarum* SKO-001 for reducing body fat and use thereof.

## Description

### [Technical Field]

The present disclosure relates to a novel strain of *Lactiplantibacillus plantarum* SKO-001 for reducing body fat and use thereof.

### [Background Art]

Body fat is an adipose tissue that makes up the body and is widely distributed under the skin, in mammary glands, and around the kidneys. It plays a role in storing fat and using it as energy, and also functions to protect internal organs and regulate body temperature. Mainly, adipose tissue is mainly composed of fat cells and water, and it is known that 1 kg of fat is converted into about 7,300 kcal of energy. Adults have about 6 to 25 kg of adipose tissue.

Although fat is crucial as an energy source and for homeostasis control, excessive accumulation of fat can lead to obesity, it is known that, among these, the accumulation of visceral fat in the abdominal cavity causes insulin resistance or hyperlipidemia in the liver, leading to abnormalities in sugar and lipid metabolism, high blood pressure, coronary artery disease, *etc.* In particular, in modern society, changes in diet have led to excessive accumulation of body fat, an increase in obesity, and an increase in various diseases resulting therefrom, and thus, the importance of reducing or regulating body fat is increasingly being emphasized along with treatments for obesity.

Treatments for obesity largely include diet-exercise therapy, surgical therapy, and drug therapy. Among these, diet-exercise therapy is a treatment method that involves low-calorie and low-fat intake and physical exercise that consumes oxygen, but this method requires patience since it must be carried out repeatedly and persistently, thus it seems to be ineffective for the general public. Surgical therapy is a method that physically removes body fat through surgical operations, and has the advantage of being effective in a short period of time. However, it is limited in use due to the burden of surgery, the lack of sustainability of the effect, and the large economic burden. Drug therapy is a method of treating or preventing obesity using drugs that decrease appetite or suppress fat absorption, but it is problematic because it carries many side effects, such as depression, yo-yo effect, *etc.*

Therefore, there is still a need for an alternative that guarantees long-term effects for reducing body fat and has fewer side effects.

### [Disclosure]

### [Technical Problem]

It is one object of the present disclosure to provide a food composition for reducing body fat, including: one or more selected from the group consisting of a *Lactiplantibacillus plantarum* strain, a culture thereof, a lysate thereof, and an extract thereof.

It is another object of the present disclosure to provide a food composition for preventing or improving obesity, including: one or more selected from the group consisting of a *Lactiplantibacillus plantarum* strain, a culture thereof, a lysate thereof, and an extract thereof.

It is still another object of the present disclosure to provide a pharmaceutical composition for preventing or treating obesity, including: one or more selected from the group consisting of a *Lactiplantibacillus plantarum* strain, a culture thereof, a lysate thereof, and an extract thereof.

It is yet another object of the present disclosure to provide a feed composition for preventing or improving obesity, including: one or more selected from the group consisting of a *Lactiplantibacillus plantarum* strain, a culture thereof, a lysate thereof, and an extract thereof.

It is even another object of the present disclosure to provide a food composition for preventing or improving a metabolic disease, including: one or more selected from the group consisting of a *Lactiplantibacillus plantarum* strain, a culture thereof, a lysate thereof, and an extract thereof.

It is further another object of the present disclosure to provide a pharmaceutical composition for preventing or treating a metabolic disease, including: one or more selected from the group consisting of a *Lactiplantibacillus plantarum* strain, a culture thereof, a lysate thereof, and an extract thereof.

It is still further another object of the present disclosure to provide a feed composition for preventing or improving a metabolic disease, including: one or more selected from the group consisting of a *Lactiplantibacillus plantarum* strain, a culture thereof, a lysate thereof, and an extract thereof.

It is still further another object of the present disclosure to provide a use of a composition, which includes one or more selected from the group consisting of a *Lactiplantibacillus plantarum* strain, a culture thereof, a lysate thereof, and an extract thereof, for reducing body fat.

It is still further another object of the present disclosure to provide a use of a composition, which includes one or more selected from the group consisting of a *Lactiplantibacillus plantarum* strain, a culture thereof, a lysate thereof, and an extract thereof, for preventing, improving or treating obesity.

It is still further another object of the present disclosure to provide a use of a composition, which includes one or more selected from the group consisting of a *Lactiplantibacillus plantarum* strain, a culture thereof, a lysate thereof, and an extract thereof, for preventing, improving or treating a metabolic disease.

It is still further another object of the present disclosure to provide a method for reducing body fat, including administering a composition, which includes one or more selected from the group consisting of a *Lactiplantibacillus plantarum* strain, a culture thereof, a lysate thereof, and an extract thereof.

It is still further another object of the present disclosure to provide a method for preventing, improving or treating obesity, including administering a composition, which includes one or more selected from the group consisting of a *Lactiplantibacillus plantarum* strain, a culture thereof, a lysate thereof, and an extract thereof.

It is still further another object of the present disclosure to provide a method for preventing, improving or treating a metabolic disease, including administering a composition, which includes one or more selected from the group consisting of a *Lactiplantibacillus plantarum* strain, a culture thereof, a lysate thereof, and an extract thereof, to a subject.

### [Technical Solution]

According to one embodiment of the present disclosure, there is provided a food composition for reducing body fat, including: one or more selected from the group consisting of a *Lactiplantibacillus plantarum* strain, a culture thereof, a lysate thereof, and an extract thereof.

According to one embodiment of the present disclosure, the strain may be a *Lactiplantibacillus plantarum* SKO-001 strain deposited with Accession No. KCTC 14816BP.

According to one embodiment of the present disclosure, the strain may have a 16S rRNA sequence represented by SEQ ID NO: 1.

According to one embodiment of the present disclosure, there is provided a food composition for preventing or improving obesity, including: one or more selected from the group consisting of a *Lactiplantibacillus plantarum* strain, a culture thereof, a lysate thereof, and an extract thereof.

According to one embodiment of the present disclosure, there is provided a pharmaceutical composition for preventing or treating obesity, including: one or more selected from the group consisting of a *Lactiplantibacillus plantarum* strain, a culture thereof, a lysate thereof, and an extract thereof.

According to one embodiment of the present disclosure, there is provided a feed composition for preventing or improving obesity, including: one or more selected from the group consisting of a *Lactiplantibacillus plantarum* strain, a culture thereof, a lysate thereof, and an extract thereof.

According to one embodiment of the present disclosure, there is provided a food composition for preventing or improving a metabolic disease, including: one or more selected from the group consisting of a *Lactiplantibacillus plantarum* strain, a culture thereof, a lysate thereof, and an extract thereof.

According to one embodiment of the present disclosure, the metabolic disease may be any one or more selected from the group consisting of obesity, diabetes, insulin resistance, hyperlipidemia, hypercholesterolemia, arteriosclerosis, hepatic steatosis, fatty liver, and cardiovascular disease.

According to one embodiment of the present disclosure, there is provided a pharmaceutical composition for preventing or treating a metabolic disease, including: one or more selected from the group consisting of a *Lactiplantibacillus plantarum* strain, a culture thereof, a lysate thereof, and an extract thereof.

According to one embodiment of the present disclosure, there is provided a feed composition for preventing or improving a metabolic disease, including: one or more selected from the group consisting of a *Lactiplantibacillus plantarum* strain, a culture thereof, a lysate thereof, and an extract thereof.

### [Advantageous Effects]

According to one embodiment of the present disclosure, the *Lactiplantibacillus plantarum* SKO-001, the probiotic strain of the present disclosure, shows excellent effects in reducing body fat and body weight, reducing cholesterol, and improving fatty liver, and thus can be effectively used for preventing, treating and improving obesity and lipid-related metabolic diseases.

### [Brief Description of Drawings]

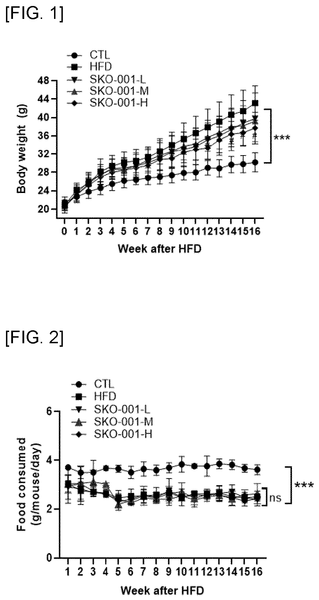

FIG. 1 shows the results of measuring changes in body weight. [The test substance was administered orally once a day for 12 weeks to C57BL/6J mice (6 weeks old, male) after 3 weeks of a high-fat diet, and the body weight was measured once a week at the same time.] For reference, the statistics are as follows: Student's t test, ***P<0.005 vs CTL, #P<0.05 vs HFD.
FIG. 2 shows the results of measuring feed intake. The test substance was orally administered once a day for 12 weeks to C57BL/6J mice (6 weeks old, male) after 3 weeks of a high-fat diet, and the feed intake was measured once a week at the same time. Here, the feed intake was calculated by subtracting the remaining feed amount from the supplied feed amount. For reference, the statistics are as follows: Student's t test, ***P<0.005 vs CTL, ns; not significant.

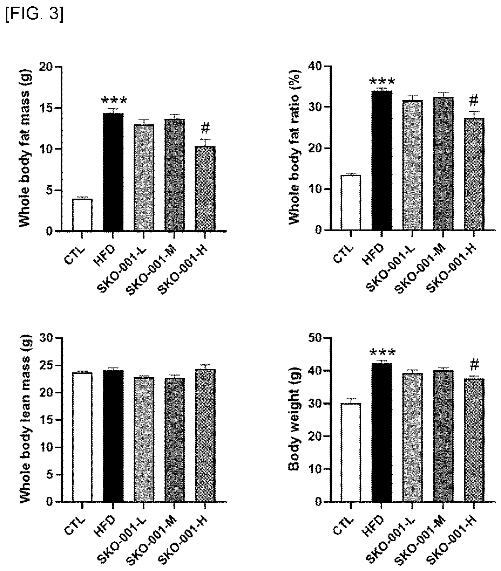

FIG. 3 shows the results of measuring body fat mass using Minispec. For reference, the statistics are as follows: Student's t test, ***P<0.005 vs CTL, #P<0.05 vs HFD.

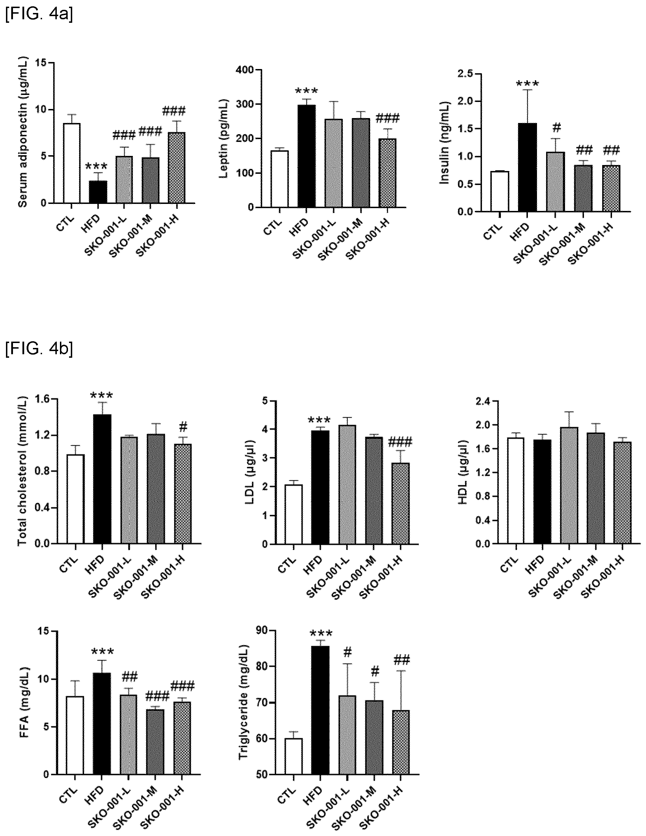

FIG. 4 shows the results of analyzing the concentrations of serum energy metabolism regulators (insulin, adiponectin, leptin) and lipids (total cholesterol, LDL-c, HDL-c, TG, FFA). FIG. 4a shows the results for the concentration of energy metabolism regulators, and FIG. 4b shows the results for the concentration of lipids. Specifically, the test substance was orally administered once a day for 12 weeks to C57BL/6J mice (6 weeks old, male) after 3 weeks of a high-fat diet, and after overnight fasting, blood was collected to measure the concentrations of serum energy metabolism regulators (insulin, adiponectin, leptin) and lipids (total cholesterol, LDL-c, HDL-c, TG, FFA). For reference, the statistics are as follows: Student's t test, ****P*<0.005 vs CTL, *#P* <0.05, *##P* <0.01, *###P* <0.005 vs HFD.

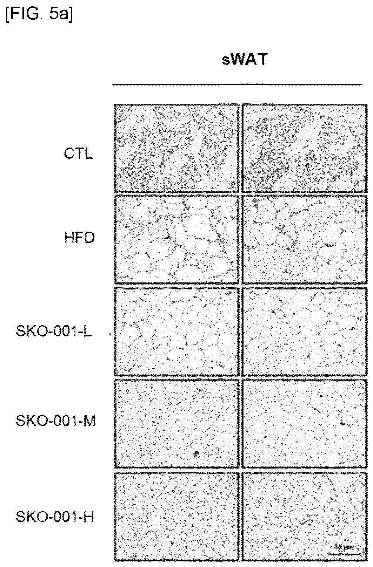

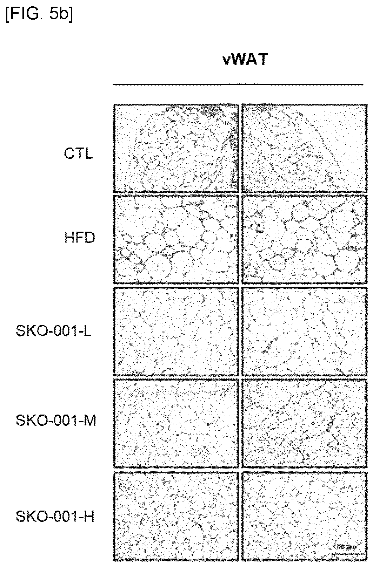

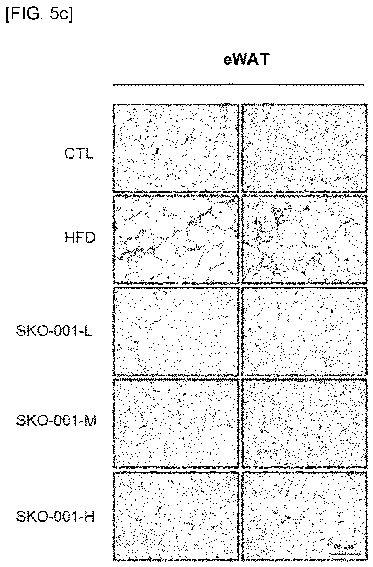

FIG. 5 shows the H&E staining results for the morphology of adipose tissues. FIG. 5a shows the results for subcutaneous adipose tissue, FIG. 5b shows the results for visceral adipose tissue, and FIG. 5c shows the results for epididymal adipose tissue. Specifically, the test substance was orally administered once a day for 12 weeks to C57BL/6J mice (6 weeks old, male) after 3 weeks of a high-fat diet, and after overnight fasting, the morphology of adipocytes was measured by H&E staining using subcutaneous adipose, visceral adipose, and epididymal adipose tissues.

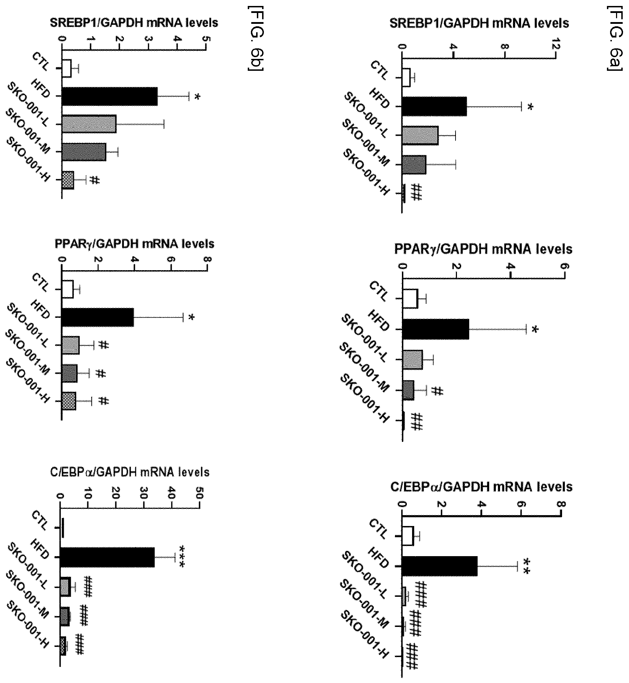

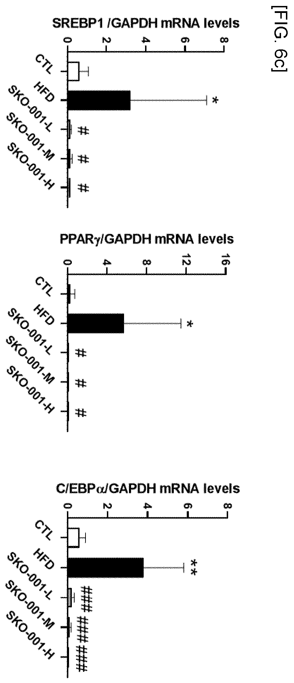

FIG. 6 shows the results of analyzing the mRNA expression of factors related to adipocyte differentiation (SREBP1, PPARγ, C/EBPα) in adipose tissue. FIG. 6a shows the results for subcutaneous adipose tissue, FIG. 6b shows the results for visceral adipose tissue, and FIG. 6c shows the results for epididymal adipose tissue. Specifically, the test substance was orally administered once a day for 12 weeks to C57BL/6J mice (6 weeks old, male) after 3 weeks of high-fat diet, and after overnight fasting, each adipose tissue was obtained, and the mRNA levels of transcription factors (SREBP1, PPARγ, C/EBPα) related to adipocyte differentiation were measured by real-time qPCR. For reference, the statistics is as follows: Student's t test, **P*<0.05, ***P*<0.01, ****P*<0.005 vs CTL, #*P*<0.05, ##*P*<0.01, ###*P*<0.005 vs HFD.

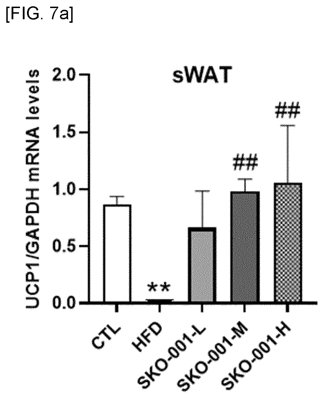

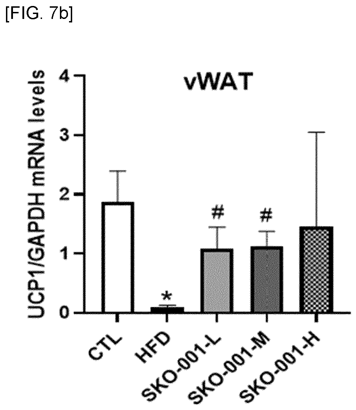

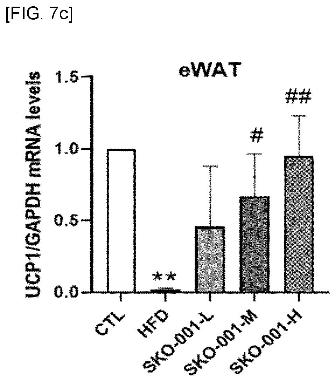

FIG. 7 shows the results of analyzing the mRNA expression of thermogenic factor (UCP1) in adipose tissue. FIG. 7a shows the results for subcutaneous adipose tissue, FIG. 7b shows the results for visceral adipose tissue, and FIG. 7c shows the results for epididymal adipose tissue. Specifically, the test substance was orally administered once a day for 12 weeks to C57BL/6J mice (6 weeks old, male) after 3 weeks of high-fat diet, and after overnight fasting, each adipose tissue was obtained, and the mRNA levels of thermogenic factor (UCP1) were measured by real-time qPCR. For reference, the statistics are as follows: Student's t test, **P*<0.05, ***P*<0.01 vs CTL, #*P*<0.05, ##*P*<0.01, ###*P*<0.005 vs HFD.

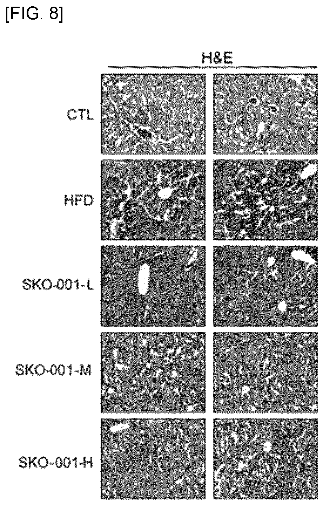

FIG. 8 shows the results of Oil Red O staining of liver tissue. Specifically, the test substance was orally administered once a day for 12 weeks to C57BL/6J mice (6 weeks old, male) after 3 weeks of a high-fat diet, and after overnight fasting, the liver tissue was obtained and fat accumulation was measured by Oil Red O staining.

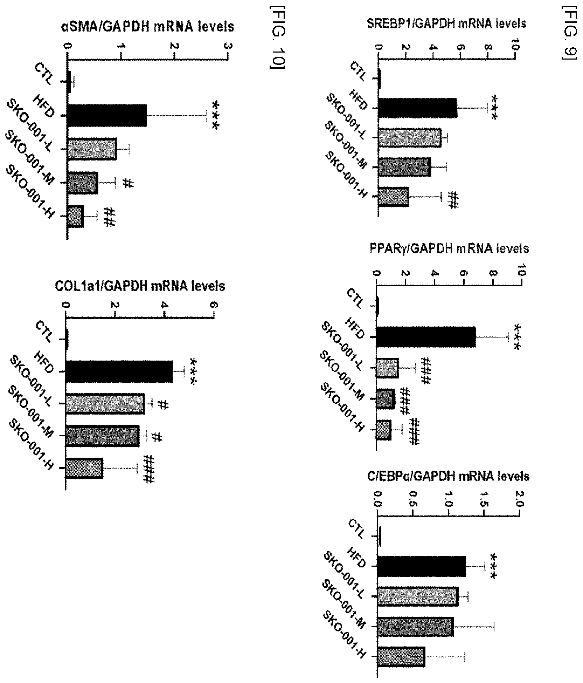

FIG. 9 shows the results of analyzing the mRNA expression of factors related to adipocyte differentiation (SREBP1, PPARγ, C/EBPα) in liver tissue. Specifically, the test substance was orally administered once a day for 12 weeks to C57BL/6J mice (6 weeks old, male) after 3 weeks of a high-fat diet, and after overnight fasting, the liver tissue was obtained, and the mRNA levels of transcription factors (SREBP1, PPARγ, C/EBPα) involved in adipocyte differentiation were measured using real-time qPCR. For reference, the statistics are as follows: Student's t test, ****P*<0.005 vs CTL, #*P*<0.05, ##*P*<0.01, ###*P*<0.005 vs HFD.
FIG. 10 shows the results of analyzing the mRNA expression of fibrosis-related factors (αSMA, Col1a1) in liver tissue. Specifically, the test substance was orally administered once a day for 12 weeks to C57BL/6J mice (6 weeks old, male) after 3 weeks of a high-fat diet, and after overnight fasting, the liver tissue was obtained, and the mRNA levels of (fibrosis-related factors (αSMA, Col1a1) were measured using real-time qPCR. For reference, the statistics are as follows: Student's t test, ****P*<0.005 vs CTL, #*P*<0.05, ##*P*<0.01, ###*P*<0.005 vs HFD.

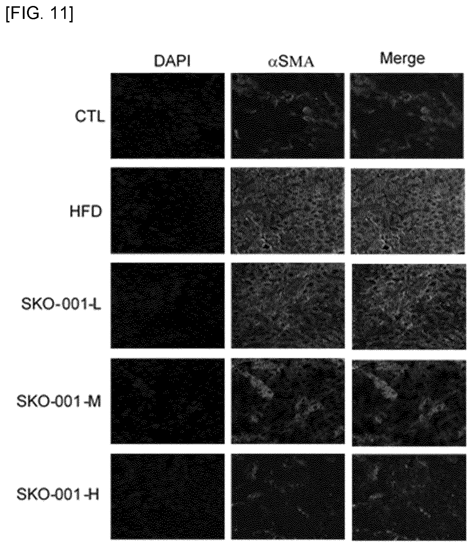

FIG. 11 shows the results of SMA immunostaining of liver tissue.

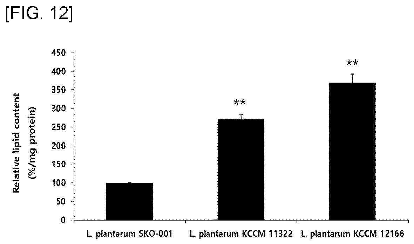

FIG. 12 shows the results of confirming the adipocyte differentiation inhibitory ability of the SKO-001 strain of the present disclosure, and KCCM11322 and KCCM12166, the homologous strains.

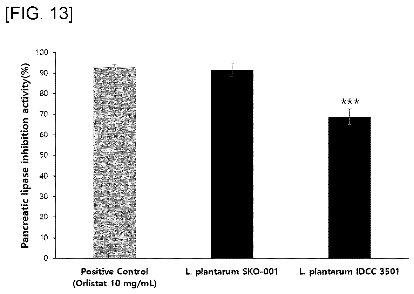

FIG. 13 shows the results of comparing the pancreatic lipase inhibitory activity of the SKO-001 strain of the present disclosure and the homologous strain IDCC 3501.

### [Detailed Description of Preferred Embodiments]

The present disclosure will be described in detail as follows. Meanwhile, each description and embodiment disclosed herein can be applied to other descriptions and embodiments, respectively. That is, all combinations of various elements disclosed herein fall within the scope of the present disclosure. Further, the scope of the present disclosure is not limited by the specific description described below.

Additionally, those of ordinary skill in the art can recognize or confirm, using only conventional experimentation, many equivalents to the particular aspects of the disclosure described herein. Furthermore, it is also intended that these equivalents be included in the present disclosure.

The present disclosure has been implemented based on the confirmation that a *Lactiplantibacillus plantarum* strain, particularly a novel strain SKO-001, has a novel use.

Hereinafter, the novel use of the *Lactiplantibacillus plantarum* strain of the present disclosure according to one embodiment of the present disclosure will be described in more detail.

One aspect according to one embodiment of the present disclosure provides a food composition for reducing body fat, including: one or more selected from the group consisting of a *Lactiplantibacillus plantarum* strain, a culture thereof, a lysate thereof, and an extract thereof.

The *Lactiplantibacillus plantarum* strain is a type of lactic acid bacterium and is a strain formerly referred to as *Lactobacillus plantarum.*

As used herein, the term *"Lactobacillus plantarum"* refers to a Gram-positive bacillus that is one of the most widely distributed lactic acid bacteria, and produces lactic acid through fermentation of arabinose, glucose, fructose, galactose, maltose, sucrose, dextran, *etc.* It is mainly isolated from dairy products, pickles, and pickled vegetables such as kimchi, tomatoes, *etc.,* especially in the latter stage of kimchi fermentation, when kimchi is fermented too much and becomes sour, it mainly grows and dominates, inhibiting the growth of other species. Typically, it is known to have excellent acid resistance and bile resistance.

The *Lactiplantibacillus plantarum* strain may be the SKO-001 strain deposited with Accession No. KCTC 14816BP, but is not limited thereto.

Additionally, the *Lactiplantibacillus plantarum* strain may have a 16S rRNA sequence represented by SEQ ID NO: 1, and may be isolated and identified from *Angelica gigas,* specifically from *Angelica gigas Nakai,* but is not limited thereto.

As used herein, the term "culture thereof" refers to a culture of the *Lactiplantibacillus plantarum* strain of the present disclosure, and may be a culture product obtained by culturing the strain of the present disclosure in a medium. For example, it may include, but is not limited to, the bacterial cells themselves obtained from the culture, or the culture supernatant obtained by filtering and centrifuging the culture.

As used herein, the term "lysate thereof" refers to a lysate of the *Lactiplantibacillus plantarum* strain of the present disclosure, and may include all useful substances present in the strain that can be released by lysis of the strain.

As used herein, the term "extract thereof" refers to an extract obtained by extracting the *Lactiplantibacillus plantarum* strain of the present disclosure using a solvent, and any known solvent may be included without limitation, and any extraction method may also be included without limitation. Additionally, the extract thereof may include an extract obtained by extracting the *Lactiplantibacillus plantarum* strain itself, or an extract obtained by extracting a culture of the strain or a lysate of the strain.

The *Lactiplantibacillus plantarum* strain of the present disclosure, a culture thereof, a lysate thereof, or an extract thereof may be included in an amount of 0.00001 to 50 wt% relative to the composition, but is not limited thereto. Specifically, when it is included in an amount less than 0.00001 wt%, the effect is insignificant, and when it exceeds 50 wt%, the increase in effect relative to the amount used is insignificant, which may be uneconomical.

As used herein, the term "body fat" refers to an adipose tissue that makes up the body, and is widely distributed under the skin, mammary glands, and around the kidneys. In one example, it may be body fat present in any one area selected from the liver, mesentery, around the kidneys and under the skin, but is not limited thereto.

The food composition is for reducing body fat, and as used herein, the term "food" may include all foods in a general sense, such as meats, sausages, breads, chocolates, candies, snacks, cookies, pizzas, instant noodles, other noodles, chewing gums, dairy products including ice cream, various soups, beverages, tea, drinks, alcoholic beverages, and vitamin complexes, *etc.*

The food composition according to the present disclosure may include a health functional food composition. In a health functional food composition, when the strain is used as an additive in a health functional food, it may be added directly or in combination with other foods or food ingredients, and may be appropriately used in accordance with methods known in the art. The amount of the active ingredient to be mixed may be appropriately determined according to the intended purpose, such as a prophylactic, health or therapeutic purpose.

Formulations of the foods include not only powder, granules, pills, tablets, and capsules, but also any general foods or beverages.

There is no particular limitation on the kind of food. Examples of foods to which the above substances may be added may include all foods in a general sense, such as meats, sausages, breads, chocolates, candies, snacks, cookies, pizzas, instant noodles, other noodles, chewing gums, dairy products including ice cream, various soups, beverages, tea, drinks, alcoholic beverages, and vitamin complexes, *etc.*

Generally, in the preparation of foods or beverages, the strain may be added in an amount of 0.0001 to 50 parts by weight or less, preferably 0.1 to 20 parts by weight or less, based on 100 parts by weight of a raw material. However, in the case of long-term ingestion intended for health or hygiene purposes or for the purpose of managing health, the amount may be less than the above-described range. The active ingredient may be used in an amount more than the above-described range since there is no problem in terms of safety, thus, it is not limited to the above range.

Among the health functional foods according to the present disclosure, beverages may contain a variety of flavoring agents or natural carbohydrates as additional ingredients as in conventional beverages. The above-described natural carbohydrates may be monosaccharides, such as glucose and fructose, disaccharides, such as maltose and sucrose, polysaccharides, such as dextrin and cyclodextrin, and sugar alcohols, such as xylitol, sorbitol, erythritol, *etc.* In addition, natural sweetening agents, such as thaumatin and *stevia* extract, and synthetic sweetening agents, such as saccharin and aspartame may be used as sweetening agents. The content of the natural carbohydrate may be in the range of about 0.01 to about 0.04 g, preferably in the range of about 0.02 to about 0.03 g, based on 100 mL of the beverage according to the present disclosure.

In addition to the above ingredients, the health functional food composition according to the present disclosure may contain various nutrients, vitamins, electrolytes, flavoring agents, coloring agents, pectic acid and salts thereof, alginic acid and salts thereof, organic acids, protective colloidal thickeners, pH adjusting agents, stabilizers, preservatives, glycerin, alcohols, and carbonating agents for carbonated drinks. Further, the health functional food composition according to the present disclosure may contain fruit flesh for the production of natural fruit juices, fruit juice beverages, and vegetable beverages. The content of such additives is not limited but may be generally selected in the range of 0.01 to 0.1 parts by weight, based on 100 parts by weight of the health functional food composition of the present disclosure.

Another aspect according to one embodiment of the present disclosure provides a food composition for preventing or improving obesity, including: one or more selected from the group consisting of a *Lactiplantibacillus plantarum* strain, a culture thereof, a lysate thereof, and an extract thereof.

The terms *"Lactiplantibacillus plantarum",* "culture thereof", "lysate thereof", "extract thereof", and "food composition" are as described above.

As used herein, the term "obesity" refers to a state where food is consumed in amounts much larger than the amount of energy required by the body, the body uses some as energy, and the rest is converted to fat and stored in the body in the form of neutral fat. Such obesity causes various chronic diseases, such as hypertension, arteriosclerosis, diabetes, *etc.*

The food composition of the present disclosure may have an intended use of preventing or improving obesity.

As used herein, the term "prevention" refers to all actions of inhibiting or delaying the symptoms of obesity by consumption or administration of one or more selected from the group consisting of the strain according to the present disclosure, a culture thereof, a lysate thereof, and an extract thereof.

As used herein, the term "improvement" refers to all actions of alleviating or beneficially changing the symptoms of obesity by consumption or administration of one or more selected from the group consisting of the strain according to the present disclosure, a culture thereof, a lysate thereof, and an extract thereof.

In one embodiment, the *Lactiplantibacillus plantarum* SKO-001 strain may reduce the levels of serum low-density lipoprotein cholesterol (LDL-cholesterol). Generally, low-density lipoprotein cholesterol is often expressed as bad cholesterol, and if the level of low-density lipoprotein cholesterol is outside the normal range, it increases the likelihood of developing arteriosclerosis, which thickens blood vessels, and the likelihood of developing brain, heart, and cardiovascular diseases may also increase, thus, it is very important to lower the level of low-density lipoprotein cholesterol.

In addition, in another embodiment, the *Lactiplantibacillus plantarum* SKO-001 strain may lower the level of serum leptin and increase the level of serum adiponectin. Leptin is a major hormone secreted by fat cells regulating appetite, and can be found in many animals. A greater amount of leptin is found in the body of obese animals, which works to reduce appetite. Meanwhile, adiponectin is a hormone secreted in greater amounts in animals with less fat than in obese animals, and is known to have an opposite effect to leptin. The novel strain of the present disclosure can prevent, improve, or treat obesity by lowering the level of serum leptin and increasing the level of adiponectin.

In still another embodiment, the strain of the present disclosure may inhibit differentiation of adipocytes and accumulation of fat, and specifically may reduce weight gain while having no effect on the decrease in appetite. In particular, unlike the conventional drug therapy for obesity treatment, which involves foods or drugs that cause a decrease in appetite or inhibit fat absorption, and have many side effects such as depression, the yo-yo effect, *etc.,* the novel strain of the present disclosure has the effect of reducing body fat and weight gain without reducing appetite, and thus can be effectively used to improve obesity without the side effects of conventional foods or drugs.

Accordingly, the present disclosure provides a food composition or a health functional food composition for reducing body fat, or preventing or improving obesity, including: the *Lactiplantibacillus plantarum* SKO-001 strain, a culture thereof, a lysate thereof, or an extract thereof.

Still another aspect according to one embodiment of the present disclosure provides a pharmaceutical composition for preventing or treating a metabolic disease, including: one or more selected from the group consisting of the *Lactiplantibacillus plantarum* strain, a culture thereof, a lysate thereof, and an extract thereof.

Yet another aspect according to one embodiment of the present disclosure provides a health functional food composition for preventing or improving a metabolic disease, including: one or more selected from the group consisting of the *Lactiplantibacillus plantarum* strain, a culture thereof, a lysate thereof, and an extract thereof.

As used herein, the term *Lactiplantibacillus plantarum* strain", "culture thereof", "lysate thereof", and "extract thereof" are as described above.

As used herein, the term "metabolic disease" collectively refers to a disease that is caused by metabolic disorders in the body, and specifically may include any one or more selected from the group consisting of obesity, diabetes, insulin resistance, hyperlipidemia, hypercholesterolemia, arteriosclerosis, hepatic steatosis, fatty liver, and cardiovascular disease, but is not limited thereto.

Herein, diabetes is meant to include insulin-dependent diabetes (type 1 diabetes) and insulin-independent diabetes (type 2 diabetes), and further, diabetes includes diabetes that occurs when the pancreas is damaged due to other diseases, such as diabetes caused by hyperthyroidism, hypoadrenocorticism, excess secretion of growth hormone or catecholamines, or gestational diabetes mellitus.

As used herein, the term "prevention" refers to all actions of inhibiting or delaying the development of a metabolic disease by administration of the pharmaceutical composition according to the present disclosure.

As used herein, the term "treatment" refers to all actions of alleviating or beneficially changing the symptoms of a metabolic disease by administration of the pharmaceutical composition according to the present disclosure.

The composition including the pharmaceutical composition of the present disclosure may contain one or more active ingredients having the same or similar function in addition to the above ingredients.

The pharmaceutical composition of the present disclosure may further contain a pharmaceutically acceptable carrier in addition to the pharmaceutical composition of the present disclosure.

In the pharmaceutical composition of the present disclosure, the novel strain, a culture thereof, a lysate thereof, or an extract thereof may be included in an amount of 0.00001 wt% to 99.99 wt% based on the total weight of the pharmaceutical composition, specifically 0.1 wt% to 90 wt%, more specifically 0.1 wt% to 70 wt%, and even more specifically 0.1 wt% to 50 wt%, but is not limited thereto, and may be variously changed depending on the condition of the subject to be administered, the type of specific disease, the stage of progression, *etc.* If necessary, it may also be included in the total amount of the pharmaceutical composition.

The pharmaceutical composition according to the present disclosure may be prepared in the form of oral formulations such as powders, granules, tablets, capsules, suspensions, emulsions, syrups, aerosols, *etc.,* and in the form of external preparations, suppositories, and sterile injection solutions, according to a conventional method, and may include a suitable carrier, excipient or diluent, which is conventionally used in the preparation of a pharmaceutical composition for formulation.

Examples of the carrier, excipient, or diluent include various compounds or mixtures, including lactose, dextrose, sucrose, sorbitol, mannitol, xylitol, erythritol, maltitol, starch, acacia rubber, alginate, gelatin, calcium phosphate, calcium silicate, cellulose, methyl cellulose, microcrystalline cellulose, polyvinyl pyrrolidone, water, methylhydroxy benzoate, propylhydroxy benzoate, talc, magnesium stearate, mineral oil, *etc.*

In the case of formulation, the formulation may be prepared using diluents or excipients such as fillers, extenders, binders, humectants, disintegrants, surfactants, *etc.,* which are conventionally used.

The solid preparations for oral administration may be prepared by mixing at least one excipient, such as starch, calcium carbonate, sucrose or lactose, gelatin, *etc.,* with the strain. Further, lubricants such as magnesium stearate and talc may also be used in addition to simple excipients.

Examples of liquid preparations for oral administration include suspensions, internal use solutions, emulsions, syrups, *etc.* In addition to a simple diluent such as water or liquid paraffin, various excipients, such as wetting agents, sweetening agents, aromatics, preservatives, *etc.,* may be included.

Examples of preparations for non-oral administration include sterile aqueous solutions, non-aqueous solvents, suspensions, emulsions, lyophilizates and suppositories. Propylene glycol, polyethylene glycol, vegetable oils such as olive oil, injectable esters such as ethyl oleate, *etc.,* may be used as non-aqueous solvents and suspensions. The basic materials of suppositories include Witepsol, macrogol, Tween 61, cacao butter, laurin butter, glycerol, gelatin, *etc.*

The desirable dose of the pharmaceutical composition according to the present disclosure varies depending on the condition and the weight of a patient, the severity of a disease, the drug form, the route, and the period of administration, and may be suitably chosen by those skilled in the art. However, in order to obtain desirable effects, the pharmaceutical composition may be administered in an amount of 0.0001 to 2,000 mg/kg, preferably 0.001 to 2,000 mg/kg per day. The dose may be administered in a single or multiple doses per day. However, the scope of the present disclosure is not limited by the dose.

The pharmaceutical composition according to the present disclosure may be administered to mammals, such as rats, mice, livestock, or humans via various routes. All modes of administration are contemplated. For example, administration may be performed orally, intrarectally, or by intravenous, intramuscular, subcutaneous, intrauterine, or intracerebroventricular injection.

As used herein, the term "subject" may refer to all animals, including humans that have obesity or metabolic disease or are likely to develop obesity or metabolic disease. These animals may be mammals such as cattle, horses, sheep, pigs, goats, camels, antelope, dogs, and cats that require treatment for similar symptoms as well as humans, but the term is not limited thereto. In addition, the subject may be a human in need of the composition of the present disclosure, or a subject other than a human, but the term is not limited thereto. Even another aspect according to one embodiment of the present disclosure provides a feed composition for preventing or improving obesity, including: one or more selected from the group consisting of the *Lactiplantibacillus plantarum* strain, a culture thereof, a lysate thereof, and an extract thereof.

Further another aspect according to one embodiment of the present disclosure provides a feed composition for preventing or improving a metabolic disease, including: one or more selected from the group consisting of the *Lactiplantibacillus plantarum* strain, a culture thereof, a lysate thereof, and an extract thereof.

The terms *"Lactiplantibacillus plantarum* strain", "culture thereof", "lysate thereof", "extract thereof", "obesity", "prevention", "improvement", and "metabolic disease" are as described above.

The feed composition according to the present disclosure may improve the health condition of animals, improve body weight gain and meat quality of livestock, and increase milk yield and immunity by replacing the existing antibiotics for animals and inhibiting the growth of harmful pathogens. The feed composition may be prepared in the form of fermented feed, blended feed, pellets, or silage.

The fermented feed may be prepared by adding various microorganism groups or enzymes in addition to the strain in order to ferment organic materials, and the blended feed may be prepared by mixing various types of general feeds with the strain. The feed in the form of a pellet may be prepared by applying heat and pressure to the blended feed, *etc.,* in a pellet machine, and the silage may be prepared by fermenting green fodder with microorganisms. A wet fermented feed may be prepared by collecting and transporting organic materials such as food waste, mixing them with an excipient for adjusting a sterilization process and moisture at a predetermined ratio, and then fermenting the mixture at a temperature suitable for fermentation for 24 hours or more, and adjusting the moisture content to approximately 70%. A fermented dry feed may be prepared by additionally subjecting the wet fermented feed to a drying process and adjusting the moisture content to 30% to 40%.

The feed composition may be used in the form of a feed additive.

Hereinafter, the present disclosure will be described in detail by way of Examples. However, these Examples are merely preferred Examples given for illustrative purposes, and thus, the scope of the present disclosure is not intended to be limited to or by these Examples.

### Example 1. Preparation of Lactiplantibacillus plantarum Strain

The *Lactiplantibacillus plantarum* SKO-001 strain of the present disclosure was isolated and identified from *Angelica gigas* Nakai (Accession No. KCTC 14816BP).

Specifically, in order to isolate the strain of the present disclosure, the roots of *Angelica gigas* Nakai were used as test substances. First, the soil stuck to the roots of *Angelica gigas* Nakai was removed and washed three times with sterilized distilled water. Thereafter, the root surface was wiped with a sterilized filter paper, and the filter paper was diluted in sterilized water and homogenized with a stomacher for 5 minutes. The homogenized test substance was diluted to an appropriate ratio, smeared on an MRS solid medium, and cultured at 37°C for 48 hours. The resulting colonies were classified by shape and color, then subjected to purification and isolation again. In order to identify the lineage of the isolated strain, 16S rRNA sequencing was performed, and as a result, it was confirmed that the isolated strain had a sequence identity of 99% with the species of *Lactiplantibacillus plantarum.*

Further, for more accurate taxonomic identification, whole-genome sequencing of the isolated strain was performed at ChunLab, Inc. (Korea). Specifically, the whole-genome sequence of the isolated strain and the whole-genome sequence of the standard strains selected from the EzBioCloud Database were compared and analyzed, and as a result, the isolated strain showed an average nucleotide identity (ANI) of 99.02% with *Lactobacillus plantarum* subsp. *plantarum* and thus was finally identified as *Lactobacillus plantarum* subsp. *plantarum* (Table 1).

Accordingly, the strain isolated from *Angelica gigas* Nakai through the above processes was named *Lactiplantibacillus plantarum* SKO-001 and deposited at the Korean Collection for Type Cultures of the Korea Research Institute of Bioscience and Biotechnology on December 8, 2021, with Accession No. KCTC 14816BP.

Meanwhile, the strain of the present disclosure was subjected to preculture and main culture for 16 to 24 hours in a culture medium containing glucose, yeast extract, peptone, polysorbate 80, magnesium sulfate, *etc.,* and then the cells were collected, suspended with a freeze-drying protectant, and freeze-dried to produce a powder sample.

### Example 2. Preparation of High-Fat Diet Mouse Obesity Model and

### Confirmation of Effect of Reducing Body Fat of Lactiplantibacillus plantarum SKO-001

### 2-1. Establishment of High-Fat Diet Mouse Model and Administration of Test Substance

The effect of reducing body fat was confirmed using *Lactiplantibacillus plantarum* SKO-001, the strain of the present disclosure.

Specifically, the animals used in the experiment were C57BL/6J mice (male, 5 weeks old), in which obesity was induced by a high-fat diet. These mice were purchased from Orient Bio and acclimatized for one week before being used in the experiment. The normal control group (CTL) was fed a normal diet, and the obesity-induced group (HFD) was fed only a high-fat diet (60% fat; Saeronbio Inc., Research Diets) for 3 weeks. The change in body weight, feed intake, and behavior was observed every week for 3 weeks, and the body weight was re-measured immediately before administration of the test substance (SKO-001) to minimize the deviation of weight per group (n=10), thus creating the administration groups as shown in Table 1 below. SKO-001 was dissolved in autoclaved tap water at low dose (5x10 ⁹ CFU/day), medium dose (1x10 ¹⁰ CFU/day), and high dose (2x10 ¹⁰ CFU/day), and administered orally to the test substance-administered groups at the same time every day for 12 weeks together with a high-fat diet.

Such conditions are summarized in Table 1 below.

**[Table 1]**

| Groups | Diet | Administered Substance | Concentration |
|---|---|---|---|
| Normal control group (CTL) | Normal diet | autoclaved tap water | N/A |
| Obesity-induced group (HFD) | High-fat diet | autoclaved tap water | N/A |
| Experimental group with low dose (SKO-001-L) | High-fat diet | *L. plantarum* SKO-001 | 5x10⁹ CFU/day |
| Experimental group with medium dose (SKO-001-M) | High-fat diet | *L. plantarum* SKO-001 | 1x10¹⁰ CFU/day |
| Experimental group with high dose (SKO-001-H) | High-fat diet | *L. plantarum* SKO-001 | 2x10¹⁰ CFU/day |

### 2-2. Analysis of Feed Intake and Change in Body Weight

During the entire experimental period, the change in body weight and feed intake was measured once a week, and the results are shown in FIGS. 1 and 2, respectively.

In the case of the change in body weight, as shown in FIG. 1, the high-fat diet group showed a significant increase in body weight gain, compared to the normal control group that consumed a normal diet, confirming that obesity was induced by the high-fat diet. Additionally, when SKO-001 of the present disclosure was orally administered once a day for 12 weeks to mice in the high-fat diet group, it was confirmed that the weight loss effect was statistically significant in the SKO-001 high-dose-administered group (HFD/SKO-001-H) from week 6 of test substance administration.

Subsequently, in the case of feed intake, as shown in FIG. 2, although the intake of general feed was significantly higher, there was no significant change in feed intake during the administration period between the feed intake of the obesity-induced group (HFD) that consumed a high-fat diet and the feed intake of the SKO-001-administered groups. These results indicate that the administration of SKO-001 does not affect appetite.

### 2-3. Measurement of Body Fat Mass

After 12 weeks of test substance administration, the body fat mass was measured non-invasively using Minispec (Mini-spec, LF-90II, Bruker Optics GmbH, Germany), and the results are shown in FIG. 3.

As a result, as shown in FIG. 3, it was confirmed that the increased body fat mass in the obesity-induced group (HFD) was significantly reduced by the administration of high-dose SKO-001, compared to the normal control group (CTL). In particular, the body fat mass and final body weight decreased in the SKO-001 high-dose-administered group (SKO-001-H), while there was no effect on the whole body lean mass. Based on the results, it can be confirmed that the SKO-001 of the present disclosure has the effect of selectively reducing only fat mass.

### 2-4. Analysis of Concentration of Serum Energy Metabolism Regulators and Lipids

In order to confirm the changes in concentrations of serum energy metabolism regulators (insulin, adiponectin, leptin) and lipids (total cholesterol, LDL-c, HDL-c, TG, FFA) due to administration of SKO-001, serum was isolated from blood collected from mice in each group after 12 weeks of test substance administration and used in the experiment. The concentration of the regulators was respectively measured using Mouse HMW & Total adiponectin ELISA (47-ADPMS-E01, ALPCO), Mouse insulin ELISA (80-INSMS-E01, ALPCO), Mouse leptin ELISA kit (KTE71186, Abbkine), Cholesterol Assay Kit - HDL and LDL/VLDL (ab65390, Abcam), CheKine^{™} Triglyceride (TG) colorimetric assay kit (KTB2200, Abbkine), Chekine^{™} Total cholesterol (TC) colorimetric assay kit (KTB2220, Abbkine), Free Fatty Acid Assay Kit (ab65341, Abcam), and the results are shown in FIG. 4.

First, in order to confirm the effect on energy metabolism regulators, the levels of serum insulin, adiponectin, and leptin were measured. As shown in FIG. 4a, it was confirmed that the insulin and leptin levels were significantly reduced by SKO-001 administration compared to the obesity-induced group (HFD). Meanwhile, the level of adiponectin, an obesity-suppressing hormone that was reduced by a high-fat diet, was significantly increased by SKO-001 administration, thus confirming that SKO-001 has an effect on obesity suppression.

Subsequently, in order to confirm whether there was an effect of improving the lipid profile, the levels of total cholesterol, LDL-c, HDL-c, TG, and FFA, which are indicators for lipids, were each measured. As a result, as shown in FIG. 4b, it was confirmed that the levels of total cholesterol, LDL cholesterol, TG, and FFA were significantly reduced in the SKO-001 high-dose-administered group (SKO-001-H), compared to the obesity-induced group (HFD). These results indicate that the novel SKO-001 strain of the present disclosure has an effect on obesity suppression.

### 2-5. Morphological Analysis of Adipose Tissue (H&E Staining)

In order to demonstrate the size of fat cells in subcutaneous adipose, visceral adipose, and epididymal adipose tissues, the tissues were extracted, washed with PBS, and fixed in 10% formalin. Thereafter, paraffin blocks were prepared, stained with hematoxylin and eosin (H&E), observed under a microscope (Zeiss Axio imager z1 fluo-microscope), and then photographed.

As a result, as shown in FIG. 5, it was confirmed that the size of fat cells increased by a high-fat diet in all three adipose tissues and was significantly reduced in a dose-dependent manner by the administration of the novel SKO-001 strain of the present disclosure, which indicates an effect of suppressing fat accumulation.

### 2-6. Confirmation of Regulation of Expression of Factors Related to Adipocyte Differentiation in Adipose Tissues

After 12 weeks of test substance administration, the changes in mRNA expression of transcription factors (SREBP1, PPARγ, C/EBPα) important for adipocyte differentiation were confirmed. In this regard, three types of adipose tissues (subcutaneous adipose tissue, visceral adipose tissue, epididymal adipose tissue) were obtained, and then RNA was extracted from the tissue using the easy-BLUE^{™} kit, cDNA was synthesized from 100 ng of RNA, and real-time qPCR was performed using the following primer sequences:
SREBP1: forward (5'- CGACTACATCCGCTTCTTGCAG-3') and reverse (5'-CCTCCATAGACACATCTGTGCC-3); PPARγ: forward (5'-GTACTGTCGGTTTCAGAAGTGCC-3') and reverse (5'ATCTCCGCCAACAGCTTCTCCT-3'); C/EBPα: forward (5'-GCAAAGCCAAGAAGTCGGTGGA-3') and reverse (5'-CCTTCTGTTGCGTCTCCACGTT-3').

As a result, as shown in FIG. 6, it was confirmed that the mRNA expression levels of SREBP1, PPARγ, and C/EBPα increased by a high-fat diet in all three types of adipose tissues and were significantly reduced by SKO-001 administration. These results suggest that the novel SKO-001 strain of the present disclosure exhibits an excellent anti-obesity activity by suppressing the expression of transcription factors (SREBP1, PPARγ, C/EBPα) that promote adipocyte differentiation.

### 2-7. Confirmation of Regulation of Expression of Thermogenic Factor (UCP1) in Adipose Tissues

In order to confirm the browning effect due to SKO-001 administration in three adipose tissues (subcutaneous adipose tissue, visceral adipose tissue, and epididymal adipose tissue), real-time qPCR was performed using the UCP1 primer after RNA extraction and cDNA synthesis from the tissues:
UCP1: forward 5'-CAAAAACAGAAGGATTGCCGAAA-3' and reverse 5'-TCTTGGACTGAGTCGTAGAGG-3'.

As a result, as shown in FIG. 7, it was confirmed that the mRNA expression of UCP1, which plays an important role in the browning effect in all three types of adipose tissues, was significantly increased by SKO-001 administration. These results suggest that the novel SKO-001 strain of the present disclosure exhibits an excellent anti-obesity activity by promoting thermogenesis.

### 2-8. Inhibitory Effect of SKO-001 on Hepatic Lipid Synthesis in Liver Tissue

Since high-fat diets are known to cause non-alcoholic liver disease, the effects of SKO-001 on the inhibition of lipid accumulation and liver fibrosis in liver tissue were evaluated, and the results are shown in FIG. 8.

As a result, as shown in FIG. 8, when the degree of lipid accumulation in liver tissue was compared through Oil Red O staining, a significantly high amount of fat globules was observed in the obesity-induced group (HFD) compared to the normal diet group (CTL). However, the HFD group that consumed SKO-001 showed a decrease in fat globules in a dose-dependent manner with SKO-001.

Additionally, the mRNA expressions of transcription factors (SREBP1, PPARγ, C/EBPα) that promote adipocyte differentiation in liver tissue were compared, and the results are shown in FIG. 9. As a result, it was confirmed that, as in the adipose tissues, the mRNA expression of SREBP1 and PPARγ increased by a high-fat diet and was significantly inhibited by SKO-001 administration.

The mRNA expressions of αSMA and Col1a1, which are markers related to liver tissue fibrosis, were compared, and the results are shown in FIG. 10. As a result, it was confirmed that αSMA and Col1a1 were significantly reduced by SKO-001 administration, thereby indicating an effect of improving liver fibrosis.

Furthermore, as a result of performing αSMA immunostaining on liver tissue, as shown in FIG. 11, the level of αSMA was increased in the obesity-induced group (HFD) compared to the normal diet group (CTL), which was consistent with the results of the real-time qPCR, and was confirmed to be decreased in a dose-dependent manner by administration of the novel SKO-001 strain of the present disclosure.

In conclusion, it can be seen that the novel SKO-001 strain of the present disclosure reduces lipid accumulation in the liver by suppressing the expression of adipocyte differentiation-promoting factors not only in adipose tissues but also in liver tissue.

### Example 3. Comparison of Effect of Reducing Body Fat between Lactiplantibacillus plantarum SKO-001, and Homologous Strains KCCM11322, KCCM12166, and IDCC 3501

In order to confirm whether the *Lactiplantibacillus plantarum* SKO-001 strain of the present disclosure exhibits excellent body fat reduction and anti-obesity activities compared to the homologous strains, the adipocyte differentiation inhibition activity and pancreatic lipase inhibition activity of the SKO-001 strain were directly compared with those of the three homologous strains (KCCM11322, KCCM12166, IDCC3501).

### 3-1. Comparison of Adipocyte Differentiation Inhibition Activity

A comparative experiment was conducted to measure the degree of differentiation inhibition of mouse adipocytes (3T3-L1) with two strains (KCCM 11322, KCCM 12166), which are two homologous strains of the *Lactiplantibacillus plantarum* SKO-001 of the present disclosure. The *Lactiplantibacillus plantarum* KCCM 11322 and KCCM 12166 (type strain) strains used as comparative strains were provided by the Korean Culture Center of Microorganisms (KCCM).

The experimental procedures were as follows:
The degree of the formation of fat globules according to the treatment with *Lactiplantibacillus plantarum* strain was confirmed by Oil Red O staining. First, 3T3-L1 cells were seeded at 8x10⁴ cells/mL in 6-well plates and differentiated for 8 days. Then, the strain lysate was treated at a 4% concentration for 8 days during differentiation. Thereafter, the cells were fixed with 4% paraformaldehyde for 30 minutes at room temperature, washed with 1X PBS, and stained with Oil Red O solution for 1 hour at room temperature. The thus-stained fat globules were washed again with 1X PBS and observed under a microscope. Subsequently, the thus-stained fat globules were extracted with 100% iso-propanol, and the absorbance was measured at 540 nm using a microplate reader. After conversion to the protein concentration of each lysate, the protein concentration of each homologous strain was confirmed when the lipid content of the SKO-001-administered group was set as 100%. For reference, the statistics are as follows: Student's t test, **p<0.01 vs SKO-001.

As a result, as shown in FIG. 12, the lipid content of KCCM 11322 and KCCM 12166, the comparative strains, was 271.1% and 369.2%, respectively, which showed a significant difference of 2.7 to 3.6 times in lipid accumulation rate compared to the SKO-001 strain of the present disclosure. Based on these results, it can be seen that the SKO-001 strain of the present disclosure exhibits significantly excellent lipid accumulation inhibition activity compared to KCCM 11322 and KCCM 12166, the homologous comparative strains, and further confirmed that it has an excellent effect of reducing body fat by inhibiting adipocyte differentiation activity of preadipocytes.

### 3-2. Pancreatic Lipase Inhibition Activity Test

The degree of pancreatic lipase inhibition activity was compared between the *Lactiplantibacillus plantarum* SKO-001 of the present disclosure and a homologous strain (IDCC 3501) isolated from kimchi.

Specifically, the pancreatic lipase inhibition activity due to the treatment with the *Lactiplantibacillus plantarum* strain was measured based on the inhibition of hydrolytic conversion of p-nitrophenyl palmitate (pNPP) to p-nitrophenyl. 10 mM pNPP and 10 mg/mL of pancreatic lipase were added with the strain culture and reacted at 37°C for 30 minutes. Thereafter, the mixed reactant was placed on ice for 10 minutes to stop the enzyme reaction. Subsequently, the reactant was centrifuged at 13,000 rpm for 1 minute to obtain 100 µL of the supernatant, and the absorbance was measured at 405 nm using a microplate reader. Orlistat was used as a positive control, and the pancreatic lipase inhibition activity was expressed as a decrease rate by comparing the absorbance values of the sample-administered group and no sample-administered group. For reference, the statistics is are as follows: Student's t test, ***p<0.001 vs SKO-001.

As a result, as shown in FIG. 13, the pancreatic lipase inhibition activities of orlistat used as a positive control and the comparative strain IDCC 3501 were confirmed to be 96.4% and 68.7%, respectively. Meanwhile, the pancreatic lipase inhibition activity of SKO-001 of the present disclosure was 92.6%, showing an inhibition effect equivalent to that of the positive control, and in particular, it showed a relatively high inhibition effect compared to the IDCC 3501 strain.

These results indicate that the SKO-001 strain of the present disclosure has a significantly excellent pancreatic lipase inhibition activity compared to the homologous strain IDCC3501, suggesting that the novel SKO-001 strain directly inhibits the activity of pancreatic lipase to reduce body fat absorption and thereby exhibits an excellent effect on reducing body fat.

From the foregoing, a skilled person in the art to which the present disclosure pertains will be able to understand that the present disclosure may be embodied in other specific forms without modifying the technical concepts or essential characteristics of the present disclosure. In this regard, the exemplary embodiments disclosed herein are only for illustrative purposes and should not be construed as limiting the scope of the present disclosure. On the contrary, the present disclosure is intended to cover not only the exemplary embodiments but also various alternatives, modifications, equivalents, and other embodiments that may be included within the spirit and scope of the present disclosure as defined by the appended claims.

## Claims

1. A food composition for reducing body fat, comprising: one or more selected from the group consisting of a *Lactiplantibacillus plantarum* strain, a culture thereof, a lysate thereof, and an extract thereof.

2. The composition of claim 1, wherein the strain is the *Lactiplantibacillus plantarum* SKO-001 strain deposited with Accession No. KCTC 14816BP.

3. The composition of claim 1, wherein the strain has a 16S rRNA sequence represented by SEQ ID NO: 1.

4. A food composition for preventing or improving obesity, comprising: one or more selected from the group consisting of a *Lactiplantibacillus plantarum* strain, a culture thereof, a lysate thereof, and an extract thereof.

5. A pharmaceutical composition for preventing or treating obesity, comprising: one or more selected from the group consisting of a *Lactiplantibacillus plantarum* strain, a culture thereof, a lysate thereof, and an extract thereof.

6. A feed composition for preventing or improving obesity, comprising: one or more selected from the group consisting of a *Lactiplantibacillus plantarum* strain, a culture thereof, a lysate thereof, and an extract thereof.

7. A food composition for preventing or improving a metabolic disease, comprising: one or more selected from the group consisting of a *Lactiplantibacillus plantarum* strain, a culture thereof, a lysate thereof, and an extract thereof.

8. The composition of claim 7, wherein the metabolic disease is any one or more selected from the group consisting of obesity, diabetes, insulin resistance, hyperlipidemia, hypercholesterolemia, arteriosclerosis, hepatic steatosis, fatty liver, and cardiovascular disease.

9. A pharmaceutical composition for preventing or treating a metabolic disease, comprising: one or more selected from the group consisting of a *Lactiplantibacillus plantarum* strain, a culture thereof, a lysate thereof, and an extract thereof.

10. A feed composition for preventing or improving a metabolic disease, comprising: one or more selected from the group consisting of a *Lactiplantibacillus plantarum* strain, a culture thereof, a lysate thereof, and an extract thereof.
